(19) Europäisches Patentamt
European Patent Office
Office européen des brevets

(11) EP 2 646 828 B1

(12) **EUROPEAN PATENT SPECIFICATION**

(45) Date of publication and mention
of the grant of the patent:
**06.04.2016 Bulletin 2016/14**

(51) Int Cl.:
***G01N 33/569*** (2006.01)

(21) Application number: **11794450.4**

(22) Date of filing: **05.12.2011**

(86) International application number:
**PCT/EP2011/071734**

(87) International publication number:
**WO 2012/072816 (07.06.2012 Gazette 2012/23)**

(54) **METHOD FOR DETERMINING THE TITRE OF VIRUSES BY USING INFECTIOUS STANDARDS**

VERFAHREN ZUR BESTIMMUNG DES TITERS VON VIREN DURCH VERWENDUNG VON INFEKTIÖSEN STANDARDSUBSTANZEN

PROCÉDÉ DÉTERMINANT LE TITRAGE DE VIRUS AU MOYEN DE NORMES D'INFECTIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.12.2010 EP 10306347**

(43) Date of publication of application:
**09.10.2013 Bulletin 2013/41**

(73) Proprietor: **Texcell, .**
**91000 Evry (FR)**

(72) Inventor: **DORANGE, Fabien**
**F-28630 Nogent Le Phaye (FR)**

(74) Representative: **Regimbeau**
**20, rue de Chazelles**
**75847 Paris Cedex 17 (FR)**

(56) References cited:
**WO-A1-2010/078229    US-A1- 2006 195 268**
**US-A1- 2008 233 561**

• **PETER T. WITKOWSKI ET AL: "Cellular impedance measurement as a new tool for poxvirus titration, antibody neutralization testing and evaluation of antiviral substances", BIOCHEMICAL AND BIOPHYSICAL RESEARCH COMMUNICATIONS, vol. 401, no. 1, 8 October 2010 (2010-10-08), pages 37-41, XP055000087, ISSN: 0006-291X, DOI: 10.1016/j.bbrc.2010.09.003 cited in the application**

• **National RNAi Core Facility: "Relative Viral Titering Using Cell Viability Assay (RIU Method)", , 22 January 2008 (2008-01-22), XP002638320, Retrieved from the Internet: URL:http://dfpcorec-p/wf/web/citenpl/citenpl.html?_url=http%3A%2F%2Frnai.genmed.sinica.edu.tw%2Ffile%2Fprotocol%2F4_1_EstimationLentivirusTiterRIUV1.pdf [retrieved on 2011-05-23] cited in the application**

• **MORI S ET AL: "A colorimetric LDH assay for the titration of infectivity and the evaluation of anti-viral activity against ortho- and paramyxoviruses.", THE TOHOKU JOURNAL OF EXPERIMENTAL MEDICINE DEC 1995 LNKD-PUBMED:8928191, vol. 177, no. 4, December 1995 (1995-12), pages 315-325, XP002638266, ISSN: 0040-8727**

• **J O GOLUB: "A single-dilution method for the estimation of LD50 titers of the psittacosis-LGV group of viruses in chick embryos.", J Immunol., 1948, pages 71-82, XP055000091, Retrieved from the Internet: URL:http://www.jimmunol.org/content/59/1/71.full.pdf#page=1&view=FitH [retrieved on 2011-05-23]**

• **Jean M Riley ET AL: "Single-Dose Assay Technique for Variola Virus", Appl. Envir. Microbiol., 1964, pages 7-9, XP055000093, Retrieved from the Internet: URL:http://aem.asm.org/cgi/reprint/12/1/7. pdf [retrieved on 2011-05-23]**

**Description**

The present invention is directed to a titration method for cytopathogenic viruses. The present invention could be applied to any infectious agents that induce an effect in cells that could be quantifiable by the use of a marker, preferably a cell viability marker, including metabolites, reporter protein, or agent's components. In particular, the present invention is based on a new method for in vitro determining the titre of cytopathogenic agent in solution by comparing the viability of the infected cell against a standard curve obtained by measuring the cell viability of monolayers or in suspension cultured cells infected with a known cytopathogenic agent stock. The invention is of special utility in pharmaceutical and biotechnological industries for cytopathogenic agent clearance studies and for the identification and optimization of anti-cytopathogenic agent compounds involved in drug development and/or in pharmaceutical compositions.

Virus titration is a method used in several fields in virology:

-    Vaccine production and gene therapy when using virus or viral vector.
-    Recombinant protein expression when using viral expression system in order to define the multiplicity of infection for maximal protein expression (baculovirus, semliki forest virus).
-    Viral clearance studies in order to estimate the inactivation/removal of viruses during manufacturing process.

The methods most commonly used for the determination of virus titre are plaque assays and endpoint dilution assays (Kaplan M. & Koprowski H., 1973; Schwartz D., 1993).

Plaques assays and end point dilution assays are gold standards methods to perform the titration of viruses. However these methods are labor intensive (preparation of the cell monolayers one day before the titration, add of agarose or CMC monolayer for plaque assays ("CMC" for carboxymethylcellulose) and are time and reagent-consuming (in general 7 days to complete, require high virus supernatants and cellular volumes to handle, and high number of replicates). They need highly trained technicians and are difficult to adapt for automatisation and high throughput.

Some improvements of these methods were assessed, such as the use of MTT (3-(4,5-Dimethylthiazol-2-yl)-2,5-diphenyltetrazolium bromide) (Mena et al, 2003) or immunodetection methods to facilitate the readings of infected versus non-infected wells (Kitts PA & Green G). But in any cases, these improvements don't overcome the major drawbacks of gold standard methods.

Several recent methods were developed in order to prevent such limitations of gold standards assays.

Due to the development of these new methods, methods were divided into two groups: cell-based assays (including plaque assays and end-point dilution assays) and direct assays. Cell-based assays are infectious assays that determine the number of infectious viruses and are also qualitative and quantitative methods, whereas direct assays determine the number of a virion component (most commonly the viral genome) that can not be directly correlated to an infectious state and is also defined as a quantitative method.

In cell-based methods, immunodetection methods using flow cytometry have been successfully applied for the determination of virus titre by detecting viral proteins in infected cells (Mulvania et al., 2004). Immunodetection assays are rapid methods when performed with a flow cytometer, but limitations are link to the use of specific antibodies for each virus that could show lot by lot variation. This method needs expensive reagent and specialized equipment, and as being recognized less sensitive than plaque assays (Lambeth et al, 2005).

Reporter genes have been incorporated in engineered viruses to titrate viruses by detecting the expression of their reporter proteins like GFP (Green Fluorescent Protein), β-galactosidase (Cha et al., 1997; Yahata et al., 2000) or resistance protein. These recombinant viruses could however suffer in their use in viral clearance studies validation for pharmaceutical process and in virucidal studies as their modifications could affect their main characteristics (structure, isoelectric charge).

Determination of virus titre was reported with measurement of cell viability by using Alamar blue in cell growth assay (Pouliquen et al., 2006). The authors define a good correlation between cell growth and baculovirus virus titre. However informations for mammalian viruses are not available and titre determination is in the limited range of 5.1E+06 and 2.8E+08 PFU/ml. The authors recommend 2 steps titration methods for out of range virus titre stock with the dilution of higher titre stocks and the use of alternative methods for lower titres.

The measurement of cell death was also used for titering viruses by recording the release of the LDH protein in the supernatant. However, the authors noticed the lack of sensitivity of this method with viruses having small lytic activity, like the human para-influenza virus type III (HPIV3) (Mori and al., 1995).

We can also cite the document Witkosski et al. (2010) and the patent document US2008:233561 (2008) which disclose the use of the impedance (also known as Real-Time Cell Electronic sensing (RT-CES) system) as cell viability marker for determining the titre of host cell population infected with a virus. This system is limited to the exclusive analysis of adherent cell lines and dependent on the cell number and cell morphology of the cell lines used in the assay.

The document issue from the National RNAi Core Facility (dated on January 22, 2008) can be also cited, This document discloses a method using a cell viability assay (relative infectious unit [RIU] method) according to the RNAi Core's instructions for monitoring the viral titration (cells staining).

Another cell based assay was reported with the estimation of virus titre based on viable cell size (Janakiraman et al., 2006). The cell size assay is a rapid method but this method could only be used with viruses that induce a size change in infected cells.

In direct methods, QPCR was used to determine viral titres (Lo and Chao, 2004). Flow cytometry was used to determine viral titres with the use of SYBR Green (asymmetrical cyanine dye used as a nucleic acid stain in molecular biology) to stain the virus particles (Shen et al., 2002).

As no fully satisfactory method was described, we developed a method that possesses the following characteristics:

- Infectious assay

- Quick

- Ease of results interpretation

- Universal methods (methods and reagents used are identical among different viruses).

- Range from 1E+02 to 1E+06 $TCID_{50}$/ml

- Automation

- High throughput

The following Table 1 presents the characteristics of actual method.

Table 1

| Method | Infectious assays | quick | Ease of results interpretation | Universal methods | Range from $10^2$ to $10^6$ TCID50/ml | automatisation | high throughput |
|---|---|---|---|---|---|---|---|
| Plaque assay | ☑ | ☐ | ☐ | ☑ | ☑ | ☐ | ☐ |
| End point dilution | ☑ | ☐ | ☐ | ☑ | ☑ | ☐ | ☐ |
| immunodetection assay / flow cytometry | ☑ | ☑ | ☑ | ☐ | ☐ | ☑ | ☑ |
| microscopy examination | ☑ | ☐ | ☐ | ☐ | ☑ | ☐ | ☐ |
| MTT in end point dilution assay | ☑ | ☐ | ☑ | ☑ | ☑ | ☐ | ☐ |
| cell viability assay / Alamar blue in cell viability assay | ☑ | ☑ | ☑ | ☐ | ☐ | ☑ | ☑ |
| cell size assay | ☑ | ☑ | ☑ | ☐ | ☑ | ☑ | ☑ |
| real time PCR | ☐ | ☑ | ☑ | ☐ | ☑ | ☑ | ☑ |
| sybrgreen assay | ☐ | ☑ | ☑ | ☑ | ☐ | ☑ | ☑ |

There is a need, therefore, to provide a method to determine the virus,load of a batch cell culture, method which can be used both for adherent or non-adherent cell lines, particularly for cell lines which can be cultured in suspension (non-adherent cells or adherent cells which have been adapted to culture in suspension).

There is also a need to provide a method to determine the virus,load of a batch cell culture, method which can be applied for all type of cytopathogenic viruses, particularly further adapted for viruses having small lytic activity.

There is also a need for monitoring methods that can give preferably more sensitive and rapid results than the sensitivity and the labor-intensive and time-consuming methods currently employed for that purpose.

This is the object of the present invention.

The present invention was developed to circumvent the limitations of actual methods as no methods possess all the characteristics needed.

The method of the present description, named "titration by comparison with infectious standards", is a viability cell based assay particularly based on the measurement of a total intracellular viability cell marker

In a first aspect the present description is directed to the determination of the infectious titre of a cytopathogenic agent solution, such as viral solution, by comparison with a standard curve of a known cytopathogenic agent (or virus) stock. In the method of the present description the step of titration is based on the determination of cytopathogenic agent titres by measuring the metabolic activities of infected cell (in monolayer conformation or in suspension, depending on the cell lines used), preferably by measuring the intracellular metabolic activities of said infected cell, more preferably by determining the intracellular concentration, quantity or activity of a cell viability marker which is associated to said intracellular metabolic activities of said infected cells, and more preferably at a given moment of a process, for example by using the intracellular cell viability marker ATP (Adenosine-5'-triphosphate) or LDH (Lactate deshydrogenase).

The present invention provides a cell viability-based method for in-process monitoring the titration of a cytopathogenic virus of cultured infected virus host cells, encompassing the steps of:

a) obtaining, from a host cell culture infected with a cytopathogenic virus, a plurality of samples for different initial concentrations of the virus containing sample used for infection of the host cells, preferably at a selected time of the cell culture;

b) determining the level of the viability of the host cells in the samples by measuring the intracellular metabolic activity of infected cells by measuring the intracellular concentration, quantity or activity of a significant cell viability parameter; and

c) comparing the measure of said parameter with a standard curve obtained in a same condition for a control host cell culture infected with a known virus stock,

d) determination of said titration of a cytopathogenic virus by comparison with said standard curve,

wherein the level of the viability of the host cells in the samples is determined by the measure of the intracellular ATP, after or simultaneously to the lysis of the virus infected host cells of the sample.

In a second aspect, the present invention relates to a method for the screening, the identification or the optimization of antiviral test compounds involved in drug development and/or in pharmaceutical compositions, as defined in appended claim 8, said method comprising the cytopathogenic agent titration method of the first aspect of the present invention.

In a third aspect, the present invention is directed to a method for evaluating the capacity of industrial process to eliminate or inactivate viruses and for the determination of their reduction factor, as defined in appended claim 9.

Use of sensitive reagents for the measure of cell viability in absorbance, fluorescence and luminescence present an accurate quantification of viable cell number. The cytopathogenic agent quantification is done by measuring cell viability and by comparing it with a cell viability standard curve of infected cell monolayer or infected cell in suspension.

For the first time, the inventors have demonstrated a correlation between the viability measured in a cell monolayer or in cells suspension and the infectious cytopathogenic agent load with a low detection limit, for example for viral infection around $1E+02$ $TCID_{50}$/ml ($TCID_{50}$ for median Tissue Culture Infective Dose (quantity of a cytopathogenic agent (virus) which will produce a cytopathic effect in 50 % of the cultures inoculated))

The reagent used is a marker of the cell viability and can be used to measure the viability of any cell line.

The method of the present invention is an universal titration method that could be applied for any human and animal viruses. This method can be easily automated and can be used in high throughput assay.

The method described in the present patent has been developed with viruses and can be extended to any transmissible agent causing cytopathogenic effect on target cells.

The method of the present invention resolves the actual methods limitations and answers to all characteristics.

- Infectious assay: Titrations are performed on sensitive cell lines which can be cultured in monolayer conformation (i.e. adherent cell lines) or in suspension (non-adherent cell lines or adherent cell lines adapted to be cultured in suspension) and also measured the infectious potential of cytopathogenic agent, such as viruses.

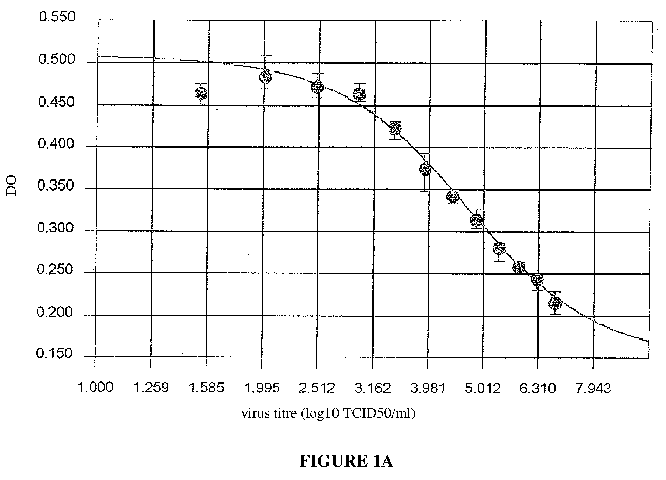

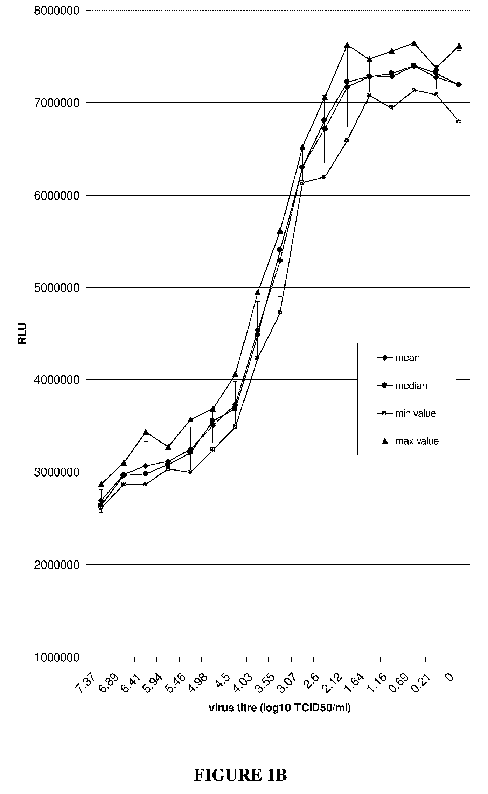

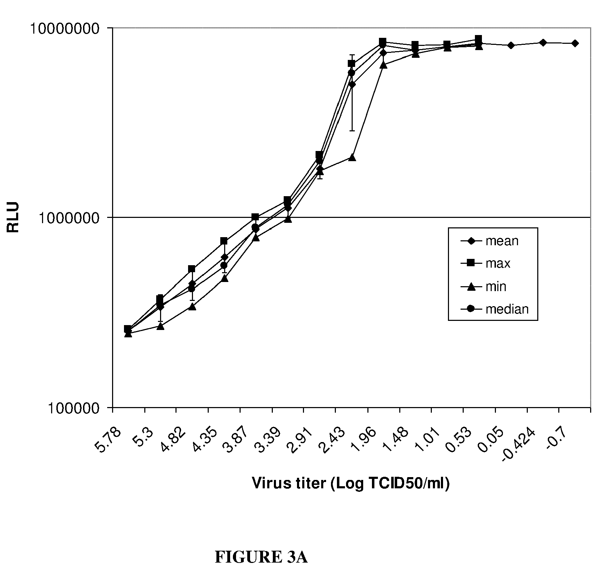

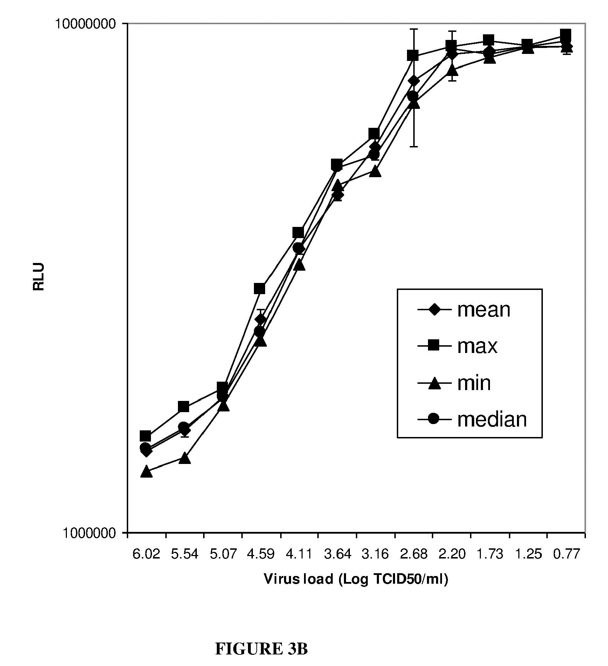

- Quick: This titration method is timeliness. For example, for viral infection, titre determination can be performed in less than four days depending of the virus instead of usually seven days in gold standard methods.
- Ease of results interpretation: Results are obtained by reading 96 wells plate on a luminometer. Data interpretation software could be link to the reader.
- Universal method: the method could be used for any cytopathogenic agent, for strong lytic or small lytic viruses, such as replicative human, animal and bacterial viruses. Figures 1A and 1B and 3 present standards curves obtained with the Minute Virus of Mice (MVM), Pseudorabies virus (PRV) and human para-influenza virus type III (HPIV3) on A9, RK13 and 293 cell lines respectively.
- Good sensitivity and large range of titration, , particularly Range from 1E+02 to 1E+06 $TCID_{50}$/ml: Figures 1A, 1B and 3 present standards curves obtained with the Minute Virus of Mice, Pseudorabies virus (PRV) and the human para-influenza virus type III on A9, RK13 and 293 cell lines respectively. The sigmoid curves obtained allow the determination of titre from approximately 1E+06 to 1E+02 $TCID_{50}$/ml for both viruses.
- Automation: Liquid handling, dilution, cells and reagents distribution and reading could be automated.
- High throughput could be easily performed as in only one 96 wells plate, several cytopathogenic agent stocks could be titrated. At least 30 wells could be attributed to standards from 1E+06 to 1E+01 $TCID_{50}$/ml for example of a known viral stock with triplicates 3 fold dilutions. This leads to 66 free wells that could be used for the titration in triplicate of 22 unknown stocks without testing different dilutions.

Thus, the present description is directed to a cell viability-based method for in-process monitoring cytopathogenic agent titration of cultured infected host cells with said cytopathogenic agent, encompassing the steps of:

a) obtaining from a host cell culture infected with said cytopathogenic agent, preferably viruses, a concentration-dependent plurality of samples;
b) determining the level of the viability of the host cells in the samples by measuring a significant cell viability parameter, preferably by determining the intracellular concentration, quantity or activity of a cell viability marker; and
c) comparing the measure of said parameter with a standard curve obtained in a same condition for a control culture of infected host cell with said cytopathogenic agent.

By "cytopathogenic agent" it is intended to designate any agent capable of infecting host cells, such as infectious virus, including bacteriophage, non-conventional transmissible agent (NCTA) and which are pathogenic for said infected host cell, preferably that are capable of causing a disease.

The term "in-process" refers in the present description to the monitoring of the parameters that are characteristic of cell and cytopathogenic agent culture, including a cytopathogenic agent infected cell culture, throughout the period of the culture. The monitoring can be periodic monitoring wherein samples are withdrawn from the culture at selected time points and for different initial concentrations of the virus containing sample, parameters such as viability are detected and measured and the parameters are plotted versus the time of the culture and the initial concentration of the cytopathogenic agent containing sample ("cytopathogenic agent containing sample" used for infection the host cells at the beginning of the culture).

In a more preferred embodiment, said cultured infected host cells are cells from adherent or non-adherent cell lines, and/or from cell lines which can be cultured in monolayer conformation and/or in suspension.

In an also more preferred embodiment, said cultured infected host cells are cells from non-adherent cell lines or from adherent cell lines which have been or which can be adapted to be cultured in suspension.

In another aspect, the present description provides a method for monitoring a cytopathogenic agent infection or for monitoring the progression of a cytopathogenic agent infection in a cell culture, said method comprising the step of:

a) infecting a host cell population with a cytopathogenic agent;
b) obtaining from said host cell culture infected with said cytopathogenic agent a concentration-dependent plurality of samples;
c) determining the level of the viability of the host cells in the samples by measuring a significant cell viability parameter, preferably by determining the intracellular concentration, quantity or activity of a cell viability marker; and
d) comparing the results or the curve obtained from said concentration-dependent plurality of samples with a standard curve obtained in a same condition for a control culture of virus infected host cell, said comparison being significant of the cytopathogenic agent infection or its progression.

In a preferred embodiment, the standard curve is obtained in a same condition for a control culture of cytopathogenic agent infected host cell in which the host cells are infected with virus or virus particles by contacting the host cells with a portion of a viral preparation having a known titre or concentration.

In a preferred embodiment, the standard curve is obtained in a same condition for a control culture of cytopathogenic agent infected host cell, wherein the cytopathogenic agent used for said control culture is the same virus, or virus having the same replication time and in which the host cells are infected with said virus or virus particles by contacting the host cells with a portion of a cytopathogenic agent preparation having a known titre or concentration.

In a preferred embodiment, the standard curve is obtained in a same condition for a control culture of said cytopathogenic agent infected host cell, wherein a range of a concentration of a cytopathogenic agent preparation having a known titre or concentration is implemented for the control culture.

In a more preferred embodiment, the standard curve is obtained by a method comprising the step of:

a) withdrawing from the culture at a selected time and for different initial concentrations of the cytopathogenic agent containing sample; and
b) determining or measuring a viability parameter of said cytopathogenic agent infected sample and plotting this parameter versus the initial virus concentration of the culture, or the initial concentration of the cytopathogenic agent containing sample used for infecting the host cells at the beginning of the culture.

In a disclosure, said cytopathogenic agent is selected from the group of virus, or virus particle. In the invention, viruses are the cytopathogenic agents.

In a preferred embodiment, this standard curve is obtained for a selected time of the culture comprised between 24 hours and 150 hours, more preferably between 48 hours and 144 hours, 72 hours $\pm$ 24 hours being the most preferred.

In a preferred embodiment, this standard curve is obtained for a range of initial virus concentration comprised between $5.10^8$ to $0,5$ $TCID_{50}$/ml, preferably $10^8$ to $10^1$, $5.10^7$ to $10^1$, or $10^7$ to $10^1$ $TCID_{50}$/ml.

In a preferred embodiment, this range of concentration is made with 3-fold dilutions of a known initial cytopathogenic agent stocks.

In a preferred embodiment, when said cytopathogenic agent is a virus, one volume, preferably 50 $\mu$l, of the virus infected sample (or standard) is added to one volume of a cell suspension, preferably said cell suspension containing between $10^3$ to $10^5$ host cells, more preferably between $5.10^3$ to $5.10^4$ host cells, $10^4 \pm 2. 10^3$ host cells being the most preferred.

In a disclosure, the step of determining the level of the viability of the host cells in the samples by measuring a significant cell viability parameter is determined by the measure of the intracellular ATP (concentration or quantity) or the intracellular LDH activity of the host cells, preferably the total intracellular ATP (concentration or quantity) or the total intracellular LDH activity of the host cells.

In the invention the step of determining the level of the viability of the host cells in the samples by measuring a significant cell viability parameter is determined by the measure of the intracellular ATP after the lysis of the cytopathogenic agent infected host cells of the sample.

The host cell lysing step can be carried out before or simultaneously the measurement or the total intracellular concentration or quantity of ATP present in the sample.

Methods that can be used for active cell lysis are known to the person skilled in the art, and have for instance been discussed (see WO 98/22588, p. 28-35). Useful methods in this respect are for example, freeze-thaw, solid shear, hypertonic and/or hypotonic lysis, liquid shear, sonication, high pressure extrusion, detergent lysis, combinations of the above, and the like. In one embodiment of the invention, the cells are lysed using at least one detergent. Use of a detergent for lysis has the advantage that it is an easy method, and that it is easily scalable.

Detergents that can be used and the way they are employed are generally known to the person skilled in the art. Detergents, as used herein, can include anionic, cationic, zwitterionic, and nonionic detergents. Exemplary detergents include but are not limited to taurocholate, deoxycholate, taurodeoxycholate, cetylpyridium, benzalkonium chloride, ZWITTERGENT-3-14®, CHAPS (3-[3-Cholamidopropyl] dimethylammoniol]-1-propanesulfonate hydrate, Aldrich), Big CHAP, Deoxy Big CHAP, Triton X-100®, Triton X-114®, C12E8, Octyl-B-D-Glucopyranoside, PLURONIC-F68®, TWEEN-20®, TWEEN-80® (CALBIOCHEM® Biochemicals), Thesit®, NP-40®, Brij-58®, octyl glucoside, and the like. It is clear to the person skilled in the art that the concentration of the detergent may be varied, for instance within the range of about 0.1%-5% (w/w). In certain embodiments the detergent is present in the lysis solution at a concentration of about 1% (w/w) (1 % +/- 0, 5 %).

The 'lysis step' (i.e. subjecting the cells containing the virus therein to lysis) as used in these embodiments, is meant to be a lysis step employing external factors, such as a detergent or by the other above-cited methods.

During the culturing of the cells wherein the cytopathogenic agent, such as virus, is in the process of propagation or infection, some cells may already lyse because of the virus presence or virus propagation and in absence of any external lysis factors.

In preferred embodiments, such lysis in the absence of external factors has occurred in less than 50%, preferably less than 40%, more preferably less than 30%, still more preferably less than 20% of the cells.

Depending of the host cells used in the present method, the lysis factor or lysis buffer can be adapted to these host cells. For example, anionic, cationic or zwitterionic detergents, such as DMSO, SDS CHAPS, Triton X®-series, such as Triton X-100® can be used for cells lysis.

In a more preferred embodiment the intracellular ATP is determined by bioluminescence.

In another disclosure, the description is directed to a method according to the present description, wherein the step of determining the level of the viability of the host cells in the samples by measuring a significant cell viability parameter is determined by the measure of the intracellular LDH activity.

In a disclosure, said intracellular LDH activity measurement is carried out by using cell-permeate tetrazolium salts, more preferably selected from the group consisting of WST-1, XTT; MTT and MTS tetrazolium salt, WST-1 being the more preferred.

As used herein, the term "host" refers to any prokaryotic or eukaryotic (e.g. mammalian, insect, yeast, plant, avian, animal, etc.) organism that is a recipient of cytopathogenic agent, such as a replicable virus or virus particle or a NCTA (Non Conventional Transmissible Agents), preferably prion.

Any cells may be used in the methods of the present invention so long as the cells may be infected by the virus of interest, preferably selected from the group consisting of cell lines or primary cells that are permissive to cytopathogenic agent infection

In the various embodiments of this method of the invention, the host cells can be selected from the group consisting of bacterial, insect, plant and animal cell

Representative host cells that may be used according to this aspect of the invention include, but are not limited to animal cells. Preferred animal host cells include mammalian cells, but reptilian or avian cells may be also used.

Particularly preferred are cells from mammalian cell lines, mouse, rat, rabbit, or Primates cell lines, more particularly human and simian, are the most preferred.

Among the cell lines host cells from cell lines that may be used according to this aspect of the invention, particularly for virus as cytopathogenic agent, they include, but are not limited to NIH3T3; 293, CHO, COS, VERO, BHK, BSC-1, Hela, rabbit embryo chondrocytes, HepG2, Hep3B, Lymphoblastoid cell lines, glial cell lines, Human T cell lines, RK-13 and A9 cell lines.

RK-13 and A9 cell lines are particularly preferred.

These and other suitable host cells are available commercially, for example, from European Collection of Cell Cultures (ECACC) or American Type Culture Collection (ATCC).

In the various embodiments of the methods of the invention, the virus can be selected from the group consisting of any viruses that could lead to a cytopathogenic effect in a cell monolayer or cell suspension leading to cell death.

Although the methods should not be construed as to be applicable solely to the strain of virus, host cells or their combination as indicated below, in the various embodiments of this method of the invention, the virus can be selected from the group comprising poxviridae, orthomyxoviridae, paramyxoviridae picornaviridae, polyomaviridae, herpesviridae, adenoviridae, parvoviridae, reoviridae, coronaviridae, flaviviridae, togaviridae, vesiculovirus, Rhabdoviridae and baculoviridae.

In a preferred embodiment, the viruses or the virus particles are selected from the group consisting of polyomavirus, adenovirus, simplex virus, varicellavirus, parvovirus, orthopoxvirus, parainfluenza virus, cardiovirus, Enterovirus, hepatovirus, Aphtovirus, paramyxovirus, vesiculovirus, alphavirus, pestivirus, flavivirus, parvovirus, alphabaculovirus.

In another preferred embodiment, the viruses belong to the family of orthomyxoviridae, in particular influenza virus. The influenza virus is selected from the group consisting of human influenza virus, avian influenza virus, equine influenza virus, swine influenza virus, feline influenza virus. Influenza virus is preferably selected in strains A, B and C. Among strains A, one can recite viruses with different subtypes of haemagglutinin and neuraminidase, such as without limitation H1N1, H2N2, H3N2, H4N2, H4N6, H5N1, H5N2, H7N7 et H9N2.

In another preferred embodiment, the viruses belong to the family of paramyxoviridae. Preferably the virus belong to the morbillivirus genus selected in the group comprising measles virus, respirovirus genus selected in the group of Sendai virus and, human para-influenza types I and III, or Pneumovirus genus such as Respiratory Syncythial virus (RSV),

In another preferred embodiment, the viruses, the related viral vectors, the viral particles and vaccines belong to the family of togaviridae. Preferably the virus is an alphavirus selected in the group comprising Sindbis virus and Semliki forest viru.

In another preferred embodiment, the viruses belong to the family of flaviviridae, particularly in the group consisting of the Genus Flavivirus in particular Dengue virus, Japanese encephalitis virus, Yellow fever virus and West Nile virus, Genus Hepacivirus (type Hepatitis C virus) and Genus Pestivirus in particular Bovine viral diarrhea virus.

In another preferred embodiment, the viruses belong to the family of coronaviridae, coronavirus genus in particular transmissible gastroenteritis virus.

In another preferred embodiment, the viruses belong to the family of Picornaviridae, preferably cardiovirus genus selected among Theiler virus, EMCV virus, enterovirus genus selected among Equine rhinovirus (ERV), Human rhinovirus (HRV), poliovirus sabin and poliovirus saukett, and hepatovirus genus such as hepatitis A virus (HAV).

In another preferred embodiment, the viruses or the viral particles belong to the family of Herpesviridae. Preferably the virus is a simplexviruses selected among the group comprising Herpes simplex virus-1 (HSV-1), Herpes simplex virus-2 (HSV-2), and varicellovirus such as Pseudorabies virus (PRV).

In another aspect, the present invention is directed to a method for the screening, the identification or the optimization of antiviral test compounds involved in drug development and/or in pharmaceutical compositions, said method comprising the step of:

A) Monitoring or determining the viral titration of cultured infected virus host cells, by the method according to the present invention and wherein the viral titration is carried out in presence and in absence of the antiviral compound which is desired to be tested;

B) Comparing the measure of the viral titration obtained and determining whether this antiviral test compound has the capacity to modulate, preferably to decrease the viral titration and, thus, the capacity to decrease or to inhibit the viral infection.

In another aspect, the present invention is directed to a method for evaluating the capacity of industrial process to eliminate or inactivate viruses and, for the determination of their reduction factor, said method comprising the step of:

A) measuring or determining by the method according to the present invention the cytopathogenic agent titration of host cell culture infected with a sample from a liquid or fluid material, said sample being taken before and after its treatment by said industrial process;

B) Comparing the measure of the cytopathogenic agent titration of host cell culture infected for said two samples in order to determine the efficiency of said industrial process to eliminate or inactivate cytopathogenic agent susceptible to be present in said material.

In another aspect, the present invention is directed to a method for evaluating the virulence of a particular virus strain compared with a standard strain of said virus, said method comprising the step of:

A) measuring or determining by the method according to the present invention the virus titration of host cell culture infected with a sample of said particular virus strain and the virus titration of host cell culture infected with a sample of said standard virus strain, in the same conditions of culture;

B) comparing the measure obtained for these two strains of virus and determining whether said particular virus strain is more virulent than the standard strain, an increase of the titre observed for the particular viral strain tested being significant of a greater virulence of that strain compared with the standard strain.

In another aspect, the present description encompasses a kit comprising reagents luciferin and luciferase for an ATP assay, or suitable reagents for an intracellular LDH assay, which further includes an instruction set (the instructions can be in another embodiment provided independently of the kit). Such instructions are characterized, in part, in that they provide a user with information related to determining the viral titre of an infected host cell culture sample. Instructions can be provided in a kit, for example, written on paper or in a computer readable form provided with the kit, or can be made accessible to a user via the internet. Such instructions can, for example, instruct a user of the kit as how to obtain the standard curve in a determined condition for a control culture of virus infected host cell and thus for practicing the cell viability-based method for in-process monitoring the viral titration of cultured infected virus host cells of the present invention.

Kits may also provide instructions for practicing a method of monitoring (progression of) a cytopathogenic agent infection in a cell or in a cell culture, for screening or identifying anti cytopathogenic agent test compounds or determining the anti-cytopathogenic agent efficiency of an industrial process

The following examples and also the figures and the legends hereinafter have been chosen to provide those skilled in the art with a complete description in order to be able to implement and use the present invention. These examples are not intended to limit the scope of what the inventor considers to be its invention, nor are they intended to show that only the experiments hereinafter were carried out.

The examples relying on the measurement of LDH activity are for illustrative purposes only.

**Legend of the figures**

**Figures 1A and 1B:** The following graph presents the standard curve obtained with the virus MVM on A9 cell line. Tests were performed with reagents WST-I (Water soluble tetrazolium, having the formula 4-[3-(4-iodophenyl)-2-(4-nitrophenyl)-2H-5-tetrazolio]-1,3-benzene disulfonate) (Roche) to measure the LDH activity (Figure 1A) and CellTiter glow (Promega) to measure the ATP activity (Figure 1B).

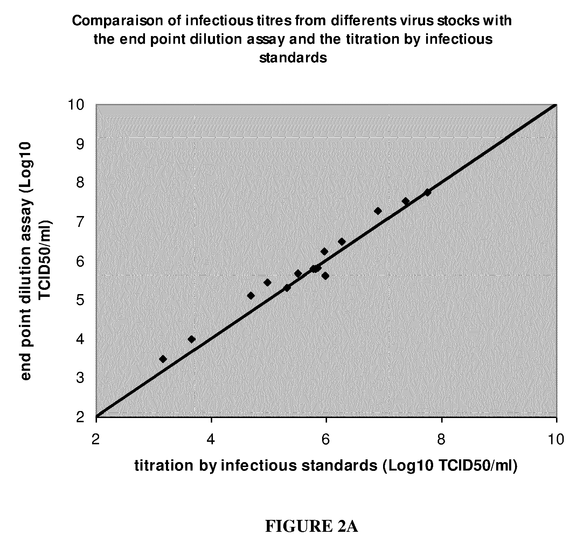

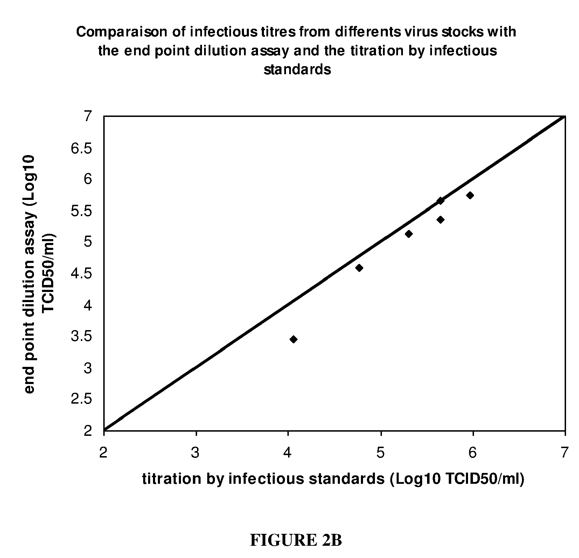

**Figures 2A and 2B:** Determination of the accuracy of the new method. Titres of different stocks were compared between end point dilution assay and the new method using the intracellular LDH (Figure 2A) and the intracellular ATP (Figure 2B) viability marker. The graphs present the results and the correlation obtained between the two methods for each intracellular viability marker.

**Figure 3A and 3B:** Determination of the universality of the method, dilutions of PRV (Figure 3A) and HPIV3 (Figure 3B) stocks were performed to prove the correlation between two other viruses load and cell viability.

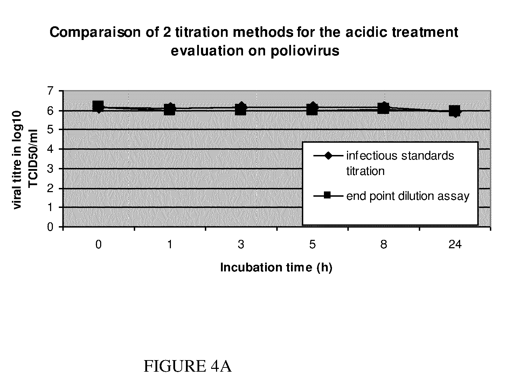

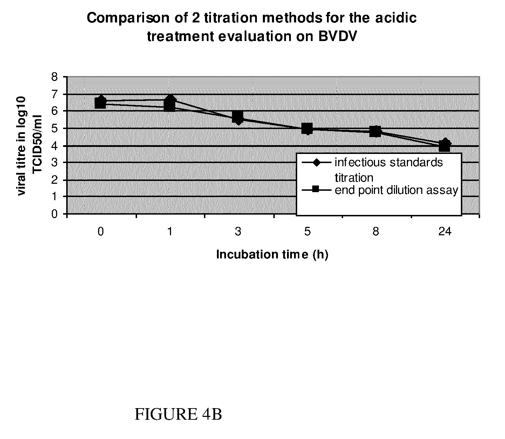

**Figures 4A and 4B:** Comparison of two titration methods for the acidic treatment evaluation on poliovirus (Figure 4A) and BVDV (Figure 4B).

## EXAMPLES

**Example 1:** Method for the determination of the viral titre

Experiments were performed in 96 wells plate. Standards from approximately $10^7$ to $10^1$ $TCID_{50}$/ml were made with 3-fold dilutions of a known virus stocks. 50 $\mu$l of the standards were added in designed wells. For unknown virus stocks, 50 $\mu$l were added in duplicate wells. Then 50 $\mu$l of a cell suspension containing $10^4$ cells were added to each wells to be tested. 96 wells plate were incubated at 37 °C with 5 % CO2 in a humidified environment. At a precise day depending on the virus tested, a cell viability test is performed to quantify the amount of a biomarker (ATP or LDH) with a plate reader (Absorbance reader, fluorimeter and luminometer).

ATP is a marker for cell viability because it's present in all metabolically active cells and the intracellular concentration declines very rapidly when the cells undergo necrosis or apoptosis. The presence of the total intracellular ATP in cells is determined by the production of light caused by the reaction of ATP (released after lysing step) with added luciferase and D-luciferin in the presence of $Mg^{2+}$ and molecular oxygen. Cells are currently lysed with detergents but can be lysed with other methods (heat or sonication). The emitted light is consequently proportional to the intracellular ATP concentration and is measured using a luminometer.

The LDH biomarker reaction (Lactate dehydrogenase) is based on the cleavage of a tetrazolium salt to a formazan-class dye by mitochondrial succinate-tetrazolium reductase in viable cells. As the cells proliferate, more WST-1 is converted to the formazan product. The quantity of formazan dye is directly related to the number of metabolically active cells, and can be quantified by measuring the absorbance at 420-480 nm ($A_{max}$ 450 nm) in a multiwell plate reader. Most of the tetrazolium salt reagents are cell-permeate and don't required lysis of the cells. By using cell-permeate tetrazolium, the lysing of the cells is not necessary to measure the total intracellular LDH.

Other cell-permeate terazolium salts, preferably water soluble, can be used such as the use of:

- the tetrazolium salt MTT (3-(4,5-dimethylthiazol-2-yl)-2,5-diphenyltetrazolium bromide);
- the tetrazolium salt XTT (3'-[1-[(phenylamino)-carbonyl]-3,4-tetrazolium]-bis(4-methoxy-6-nitro)benzene-sulfonic acid hydrate); and
- the tetrazolium salt MTS (3-(4,5-dimethylthiazol-2-yl)-5-(3-carboxymethoxyphenyl)-2-(4-sulfophenyl)-2H-tetrazolium).

A typical result shows a sigmoid curve or a polynomial curve that allow the determination of the titre between 1E+02 to 1E+06 $TCID_{50}$/ml (Figures 1A, 1B. 3A and 3B). The sigmoid function is defined as follows:

$$MES(Co) = D + (A-D) / (1 + (C0/C)B),$$

The parameter A and D describes the upper and lower limit of the sigmoid shape.

C is equal to the concentration at the turning point of the curve.

The parameter B is equivalent to the slope of the curve at the turning point of the shape.

The limit of detection is volume dependant and in this case it corresponds to 100 $TCID_{50}$/ml, or 5 $TCID_{50}$/ 50$\mu$l, or 3.5 infectious unit / tested volume that is very close to the maximal limit of detection defined as 1 infectious unit/ tested volume).

For higher titre, dilution of the stock should be done.

**Example 2:** Accuracy of the method

In order to demonstrate the accuracy of the new method, different MVM (murine minute virus) virus stocks were titrated both by the new methods and the end point dilution assay (Figures 2A and 2B). The comparison of the titre obtained by the 2 methods show a linear relationship with titres concentrating around the straight line y=x with y defined as the end point dilution assay and x defined as the new method.

**Exemple 3:** Use of the method for viral clearance studies.

In order to demonstrate the utility of the method in viral clearance studies, we evaluate the effect of an acidic treatment on two distinct viruses (a highly resistant virus poliovirus and a virus with moderate resistance BVDV (Bovine Viral Diarrhoea Virus)).
24 h time-course experiments were performed on poliovirus (Figure 4A) and BVDV (Figure 4B) in an acidic solution (pH 3). Titration were performed with the new method "titration by comparison with infectious standard" and compared with titres obtained with the dilution limit method.
Similar titres were obtained with both methods, suggesting the validity of using this new method in clearance studies.

**References:**

Cha HJ, Pham MQ, Rao G, Bentley WE.Expression of green fluorescent protein in insect larvae and its application for heterologous protein production. Biotechnol Bioeng. 1997 Nov 5;56(3):239-47.

Janakiraman V, Forrest WF, Seshagiri S.Estimation of baculovirus titer based on viable cell size.Nat Protoc. 2006;1(5):2271-6.

Kaplan M. and Koprowski H. (Ed) in: Laboratory Techniques in Rabies. Third edition. Edited by World Health Organization, Geneva, 1973.

Kitts PA, Green G. An immunological assay for determination of baculovirus titers in 48 hours. Anal Biochem. 1999 Mar 15;268(2):173-8.

C. R. Lambeth, L. J. White, R. E. Johnston, and A. M. de Silva. Flow Cytometry-Based Assay for Titrating Dengue Virus. Journal of Clinical Microbiology, July 2005, p. 3267-3272, Vol. 43, No.

Lo HR and Chao YC. Rapid titer determination of baculovirus by quantitative real-time polymerase chain reaction. Biotechnol Prog. 2004 Jan-Feb;20(1):354-60.

Mena JA, Ramírez OT, Palomares LA. Titration of non-occluded baculovirus using a cell viability assay. Biotechniques. 2003 Feb;34(2):260-2, 264.

Mori S, Watanabe W, Shigeta S. A colorimetric LDH assay for the titration of infectivity and the evaluation of antiviral activity against ortho- and paramyxoviruses. The Tohoku Journal of Experimental Medecine, December 1995.

Pouliquen Y, Kolbinger F, Geisse S, Mahnke M. Automated baculovirus titration assay based on viable cell growth monitoring using a colorimetric indicator.Biotechniques. 2006 Mar;40(3):282, 284, 286 passim.

Schwartz D. Méthodes statistiques a l'usage des médecins et des biologistes. Flammarion Médecine-Sciences (4ème édition), 1993.

Shen CF, Meghrous J, Kamen A.Quantitation of baculovirus particles by flow cytometry.J Virol Methods. 2002 Sep;105(2):321-30.

Thera Mulvania, Brooks Hayes and David Hedin. A Flow Cytometric Assay for Rapid, Accurate Determination of Baculovirus Titers. Bioprocess. Journal, may/june 2004. Yahata T, Andriole S, Isselbacher KJ, Shioda T.Estimation of baculovirus titer by beta-galactosidase activity assay of virus preparations.Biotechniques. 2000 Aug;29(2):214-5.

Witkosski P.T. et al. Biochemical and Biophysical Research Communication, 401, 37-41, 2010.

**Claims**

1.  A cell viability-based method for in-process monitoring the titration of a cytopathogenic virus of cultured infected virus host cells, encompassing the steps of:

    a) obtaining, from a host cell culture infected with a cytopathogenic virus, a plurality of samples for different initial concentrations of the virus containing sample used for infection of the host cells, preferably at a selected time of the cell culture;

b) determining the level of the viability of the host cells in the samples by measuring the intracellular metabolic activity of infected cells by measuring the intracellular concentration, quantity or activity of a significant cell viability parameter; and

c) comparing the measure of said parameter with a standard curve obtained in a same condition for a control host cell culture infected with a known virus stock,

d) determination of said titration of a cytopathogenic virus by comparison with said standard curve,

wherein the level of the viability of the host cells in the samples is determined by the measure of the intracellular ATP, after or simultaneously to the lysis of the virus infected host cells of the sample.

2. A method according to claim 1, wherein the measure of the intracellular ATP is a bioluminescence measurement.

3. The method according to claim 1or 2, wherein said host cells are cells selected from the group consisting of cell lines or primary cells that are permissive to virus infection.

4. The method according to one of claims 1 to 3, wherein said host cells are cells selected from the group consisting of animal cells, preferably mammalian cells

5. The method according to one of claims 1 to 4, wherein said host cells are cells selected from the group consisting of RK-13 and A9 cell lines.

6. The method according to one of claims 1 to 5, wherein said virus or virus particles are selected from the group consisting of any viruses that lead to a cytopathogenic effect in a cell monolayer and/or in suspension.

7. The method according to one of claims 1 to 6, wherein said virus or virus particles are selected from the group consisting of adenovirus, herpes simplex virus, parvovirus, Epstein Barr virus, parainfluenza virus, parvovirus, bovine viral diarrhea virus, sindbis virus, baculovirus, pseudorabies virus, hepatitis A virus, West nile virus and vaccinia virus.

8. A method for the screening, the identification or the optimization of antiviral test compounds involved in drug development and/or in pharmaceutical compositions, said method comprising the step of:

A) Monitoring the viral titration of cultured infected virus host cells, by the method according to one of claims 1 to 7, wherein the viral titration is carried out in presence and in absence of the antiviral test compound;

B) Comparing the measure of the viral titration obtained and determining whether this antiviral test compound has the capacity to decrease or to inhibit the viral infection.

9. A method for studies for evaluating the capacity of industrial process to eliminate or inactivate viruses and for the determination of their reduction factor said method comprising the step of:

A) measuring or determining by the method according to one of claims 1 to 7, the viral titration of host cell culture infected with a sample from a liquid, vapour or solid material, said sample being taken before and after its treatment by said industrial process;

B) Comparing the measure of the viral titration of host cell culture infected for said two samples in order to determine the efficiency of said industrial process to eliminate or inactivate viruses susceptible to be present in said material.

10. A method for evaluating the virulence of a particular virus strain compared with a standard strain of said virus, said method comprising the step of:

A) measuring or determining by the method according to one of claims 1 to 7, the viral titration of host cell culture infected with a sample of said particular virus strain and the virus titration of host cell culture infected with a sample of said standard virus strain, in the same conditions of culture;

B) comparing the measure of the titration obtained for these two strains of virus and determining whether said particular virus strain is more virulent than the standard strain, an increase of the titre observed for the particular viral strain tested being significant of a greater virulence of that strain compared with the standard strain.

**Patentansprüche**

1. Zellviabilität-basiertes Verfahren zur Überwachung im Prozess der Titration eines cytopathogenen Virus von kultivierten infizierten Viruswirtszellen, umfassend die folgenden Schritte:

   a) Erhalten, von einer Wirtszellenkultur, die mit einem cytopathogenen Virus infiziert ist, einer Vielzahl von Proben für verschiedene Ausgangskonzentrationen der Virus-enthaltenden Probe, die für die Infektion der Wirtszellen, vorzugsweise zu einer ausgewählten Zeit der Zellkultur, verwendet wird;
   b) Bestimmen des Niveaus der Viabilität der Wirtszellen in den Proben durch Messung der intrazellulären Stoffwechselaktivität von infizierten Zellen durch Messen der intrazellulären Konzentration, Menge oder Aktivität eines signifikanten Zellviabilitätsparameters; und
   c) Vergleichen der Messung des Parameters mit einer Standardkurve, die in einem gleichen Zustand für eine Kontroll-Wirtszellkultur erhalten wird, die mit einem bekannten Virusstamm infiziert ist,
   d) Bestimmen der Titration eines cytopathogenen Virus durch Vergleich mit der Standardkurve,

   wobei das Niveau der Viabilität der Wirtszellen in den Proben durch die Messung des intrazellulären ATP bestimmt wird, nach oder gleichzeitig zur Lyse der Virus-infizierten Wirtszellen der Probe.

2. Verfahren nach Anspruch 1, wobei die Messung des intrazellulären ATP eine Biolumineszenzmessung ist.

3. Verfahren nach Anspruch 1 oder 2, wobei die Wirtszellen Zellen sind, ausgewählt aus der Gruppe, bestehend aus Zelllinien oder primären Zellen, die empfänglich für eine Virusinfektion sind.

4. Verfahren nach einem der Ansprüche 1 bis 3, wobei die Wirtszellen Zellen sind, ausgewählt aus der Gruppe bestehend aus tierischen Zellen, vorzugsweise Säugetierzellen.

5. Verfahren nach einem der Ansprüche 1 bis 4, wobei die Wirtszellen Zellen sind, ausgewählt aus der Gruppe, bestehend aus RK-13- und A9-Zelllinien.

6. Verfahren nach einem der Ansprüche 1 bis 5, wobei das Virus oder die Viruspartikel ausgewählt sind aus der Gruppe, bestehend aus allen Viren, die zu einer cytopathogenen Wirkung in einer Zellmonoschicht und/oder in Suspension führen.

7. Verfahren nach einem der Ansprüche 1 bis 6, wobei das Virus oder die Viruspartikel ausgewählt sind aus der Gruppe, bestehend aus Adenovirus, Herpes simplex-Virus, Parvovirus, Epstein-Barr-Virus, Parainfluenzavirus, Parvovirus, Rinder-Virusdiarrhoe-Virus, Sindbis-Virus, Baculovirus, Pseudorabies-Virus, Hepatitis A-Virus, West-Nil-Virus und Vaccinia-Virus.

8. Verfahren zum Screening, Identifizieren oder Optimieren von antiviralen Testverbindungen, die an der Arzneimittelentwicklung und/oder an pharmazeutischen Zusammensetzungen beteiligt sind, wobei das Verfahren den folgenden Schritt umfasst:

   A) Überwachen der viralen Titration von kultivierten infizierten Virus-Wirtszellen durch das Verfahren nach einem der Ansprüche 1 bis 7, wobei die virale Titration in Anwesenheit und in Abwesenheit der antiviralen Testverbindung durchgeführt wird;
   B) Vergleichen der Messung der erhaltenen viralen Titration und Bestimmen, ob diese antivirale Testverbindung die Fähigkeit besitzt, die virale Infektion zu verringern oder zu hemmen.

9. Verfahren für Studien zur Bewertung der Fähigkeit eines industriellen Prozesses, Viren zu beseitigen oder zu inaktivieren, und zur Bestimmung ihres Reduktionsfaktors,
   wobei das Verfahren den folgenden Schritt umfasst:

   A) Messen oder Bestimmen, durch das Verfahren nach einem der Ansprüche 1 bis 7, der viralen Titration der Wirtszellenkultur, die mit einer Probe aus einer Flüssigkeit, einem Dampf oder einem Feststoff infiziert ist, wobei die Probe vor und nach ihrer Behandlung durch den industriellen Prozess entnommen wird;
   B) Vergleichen der Messung der viralen Titration der infizierten Wirtszellenkultur auf die zwei Proben, um die Effizienz des industriellen Prozesses zu bestimmen, um Viren zu beseitigen oder zu inaktivieren, die in dem Material vorhanden sein können.

**10.** Verfahren zur Bewertung der Virulenz eines bestimmten Virusstamms im Vergleich zu einem Standardstamm des Virus, wobei das Verfahren den folgenden Schritt umfasst:

A) Messen oder Bestimmen, durch das Verfahren nach einem der Ansprüche 1 bis 7, der viralen Titration der infizierten Wirtszellenkultur mit einer Probe des bestimmten Virusstamms und der Virustitration der infiziereten Wirtszellenkultur mit einer Probe des Standardvirusstamms unter den gleichen Kulturbedingungen;
B) Vergleichen der Messung der Titration, die für diese zwei Virusstämme erhalten wurde, und Bestimmen, ob der bestimmte Virusstamm virulenter als der Standardstamm ist, wobei eine Erhöhung des Titers, die für den bestimmten Virusstamm beobachtet wird, signifikant für eine größere Virulenz des Stamms verglichen mit dem Standardstamm ist.

## Revendications

**1.** Méthode basée sur la viabilité des cellules pour le contrôle en cours de procédé du titrage d'un virus cytopathogène d'une culture de cellules hôtes infectées par un virus, englobant les étapes de :

a) obtention, à partir d'une culture de cellules hôtes infectées par un virus cytopathogène, d'une pluralité d'échantillons de différentes concentrations initiales de l'échantillon contenant le virus utilisé pour l'infection des cellules hôtes, de préférence à un moment sélectionné de la culture cellulaire ;
b) détermination du niveau de viabilité des cellules hôtes dans les échantillons en mesurant l'activité métabolique intracellulaire de cellules infectées en mesurant la concentration intracellulaire, la quantité ou l'activité d'un paramètre de viabilité des cellules significatif ; et
c) comparaison de la mesure dudit paramètre à une courbe étalon obtenue dans les mêmes conditions pour une culture témoin de cellules hôtes infectées avec un stock viral connu,
d) détermination dudit titrage d'un virus cytopathogène par comparaison à ladite courbe étalon,

dans laquelle le niveau de viabilité des cellules hôtes dans les échantillons est déterminé par la mesure de l'ATP intracellulaire, après ou simultanément à la lyse des cellules hôtes infectées par le virus de l'échantillon.

**2.** Méthode selon la revendication 1, dans laquelle la mesure de l'ATP intracellulaire est une mesure de bioluminescence.

**3.** Méthode selon la revendication 1 ou 2, dans laquelle lesdites cellules hôtes sont des cellules sélectionnées dans le groupe consistant en des lignées cellulaires ou des cellules primaires qui sont permissives à une infection par un virus.

**4.** Méthode selon l'une des revendications 1 à 3, dans laquelle lesdites cellules hôtes sont des cellules sélectionnées dans le groupe consistant en des cellules animales, de préférence des cellules de mammifère.

**5.** Méthode selon l'une des revendications 1 à 4, dans laquelle lesdites cellules hôtes sont des cellules sélectionnées dans le groupe consistant en les lignées cellulaires RK-13 et A9.

**6.** Méthode selon l'une des revendications 1 à 5, dans laquelle ledit virus ou lesdites particules de virus est (sont) sélectionné (es) dans le groupe consistant en tous virus qui mènent à un effet cytopathogène dans une monocouche de cellules et/ou en suspension.

**7.** Méthode selon l'une des revendications 1 à 6, dans laquelle ledit virus ou lesdites particules de virus sont sélectionnés dans le groupe consistant en l'adénovirus, le virus simplex de l'herpès, le parvovirus, le virus d'Epstein Barr, le virus paragrippal, le parvovirus, le virus de la diarrhée virale bovine, le virus Sindbis, le baculovirus, le virus de la pseudorage, le virus de l'hépatite A, le virus du Nil occidental et le virus de la vaccine.

**8.** Méthode pour le criblage, l'identification ou l'optimisation de composés d'essai antiviraux impliqués dans le développement de médicament et/ou des compositions pharmaceutiques, ladite méthode comprenant les étapes de :

A) contrôle du titrage viral d'une culture de cellules hôtes infectées par un virus, par la méthode selon l'une des revendications 1 à 7, dans lequel le titrage viral est réalisé en présence et en l'absence du composé d'essai antiviral ;

B) comparaison de la mesure du titrage viral obtenu et détermination visant à savoir si ce composé d'essai antiviral a la capacité de diminuer ou d'inhiber l'infection virale.

9. Méthode d'étude pour l'évaluation de la capacité d'un procédé industriel pour éliminer ou inactiver des virus et pour la détermination de leur facteur de réduction, ladite méthode comprenant les étapes de :

A) mesure ou détermination par la méthode selon l'une des revendications 1 à 7, du titrage viral d'une culture de cellules hôtes infectées par un échantillon provenant d'un matériau liquide, vapeur ou solide, ledit échantillon étant prélevé avant et après son traitement par ledit procédé industriel ;
B) comparaison de la mesure du titrage viral de la culture de cellules hôtes infectées, pour lesdits deux échantillons afin de déterminer l'efficacité dudit procédé industriel pour éliminer ou inactiver des virus susceptibles d'être présents dans ledit matériau.

10. Méthode pour l'évaluation de la virulence d'une souche de virus particulière en comparaison d'une souche de référence dudit virus, ladite méthode comprenant les étapes de :

A) mesure ou détermination par la méthode selon l'une des revendications 1 à 7, du titrage viral de la culture de cellules hôtes infectées par un échantillon de ladite souche de virus particulière et du titrage viral de la culture de cellules hôtes infectées par un échantillon de ladite souche virale de référence, dans les mêmes conditions de culture ;
B) comparaison de la mesure du titrage obtenu pour ces deux souches de virus et détermination visant à savoir si ladite souche de virus particulière est plus virulente que la souche de référence, une augmentation du titre observé pour la souche virale particulière testée étant significative d'une plus grande virulence de cette souche en comparaison de la souche de référence.

FIGURE 1A

**FIGURE 1B**

Comparaison of infectious titres from differents virus stocks with
the end point dilution assay and the titration by infectious
standards

FIGURE 2A

Comparaison of infectious titres from differents virus stocks with the end point dilution assay and the titration by infectious standards

**FIGURE 2B**

**FIGURE 3A**

**FIGURE 3B**

**Comparaison of 2 titration methods for the acidic treatment evaluation on poliovirus**

FIGURE 4A

**Comparison of 2 titration methods for the acidic treatment evaluation on BVDV**

FIGURE 4B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2008233561 A
- WO 9822588 A

### Non-patent literature cited in the description

- **CHA HJ ; PHAM MQ ; RAO G ; BENTLEY WE.** Expression of green fluorescent protein in insect larvae and its application for heterologous protein production. *Biotechnol Bioeng.,* 05 November 1997, vol. 56 (3), 239-47
- **JANAKIRAMAN V ; FORREST WF ; SESHAGIRI S.** Estimation of baculovirus titer based on viable cell size. *Nat Protoc.,* 2006, vol. 1 (5), 2271-6
- Laboratory Techniques in Rabies. 1973
- **KITTS PA ; GREEN G.** An immunological assay for determination of baculovirus titers in 48 hours. *Anal Biochem.,* 15 March 1999, vol. 268 (2), 173-8
- **C. R. LAMBETH ; L. J. WHITE ; R. E. JOHNSTON ; A. M. DE SILVA.** Flow Cytometry-Based Assay for Titrating Dengue Virus. *Journal of Clinical Microbiology,* July 2005, vol. 43, 3267-3272
- **LO HR ; CHAO YC.** Rapid titer determination of baculovirus by quantitative real-time polymerase chain reaction. *Biotechnol Prog.,* January 2004, vol. 20 (1), 354-60
- **MENA JA ; RAMÍREZ OT ; PALOMARES LA.** Titration of non-occluded baculovirus using a cell viability assay. *Biotechniques,* February 2003, vol. 34 (2), 260-2, 264
- **MORI S ; WATANABE W ; SHIGETA S.** A colorimetric LDH assay for the titration of infectivity and the evaluation of anti-viral activity against ortho- and paramyxoviruses. *The Tohoku Journal of Experimental Medecine,* December 1995
- **POULIQUEN Y ; KOLBINGER F ; GEISSE S ; MAHNKE M.** Automated baculovirus titration assay based on viable cell growth monitoring using a colorimetric indicator. *Biotechniques,* March 2006, vol. 40 (3), 282, , 284, , 286
- Méthodes statistiques a l'usage des médecins et des biologistes. **SCHWARTZ D.** Flammarion Médecine-Sciences. 1993
- **SHEN CF ; MEGHROUS J ; KAMEN A.** Quantitation of baculovirus particles by flow cytometry. *J Virol Methods,* September 2002, vol. 105 (2), 321-30
- **THERA MULVANIA ; BROOKS HAYES ; DAVID HEDIN.** A Flow Cytometric Assay for Rapid, Accurate Determination of Baculovirus Titers. *Bioprocess. Journal,* May 2004
- **YAHATA T ; ANDRIOLE S ; ISSELBACHER KJ ; SHIODA T.** Estimation of baculovirus titer by beta-galactosidase activity assay of virus preparations. *Biotechniques.,* August 2000, vol. 29 (2), 214-5
- **WITKOSSKI P.T. et al.** *Biochemical and Biophysical Research Communication,* 2010, vol. 401, 37-41